# EUROPÄISCHE PATENTANMELDUNG

(11) **EP 1 733 619 A1**
(43) Veröffentlichungstag der Anmeldung: **20.12.2006**
(21) Anmeldenummer: 06013488.9
(22) Anmeldetag: 30.08.2002
(51) Int. Cl.: A01N 63/00, A61K 38/48

(54) **Verwendung eines Wirkstoffes zur Denaturierung des Keratins von Tierhaaren**

(30) Priorität: 28.08.2002 WO PCT/EP02/09598
(62) Teilanmeldung aus: 02807704.8
(71) Anmelder: KAPITZ, Carl-Heinz, 76709 Kronau (DE); Knapp, Gerhard, 68519 Viernheim (DE)
(72) Erfinder: KAPITZ, Carl-Heinz, 76709 Kronau (DE); Knapp, Gerhard, 68519 Viernheim (DE)
(74) Vertreter: Mierswa, Klaus

(57) **Zusammenfassung**

Die Erfindung betrifft die Verwendung eines Wirkstoffes zur Denaturierung des Keratins von Tierhaaren, wobei als Wirkstoff ein solches Enzym verwendet wird, welches die Proteinketten des Keratins von Tierhaaren in Oligopeptide, Mono-, Di- oder Tripeptide, abzubauen bzw. zu denaturieren imstande ist, welche Abbauprodukte nicht mehr allergisierend auf den Menschen wirken. Als Wirkstoff wird eines der Enzyme Auxillase, Alcalase, Bromelain, alpha-Chymotrypsin, Collagenase, Pepsin, Pronase, Pancreatin, Thrombin oder Trypsin, verwendet.

## Beschreibung

### Technisches Gebiet:

Die Erfindung betrifft die Verwendung eines Wirkstoffes zur Denaturierung des Keratins von Tierhaaren, wobei als Wirkstoff ein solches Enzym verwendet wird, welches die Proteinketten des Keratins von Tierhaaren in Oligopeptide, Mono-, Di- oder Tripeptide, abzubauen bzw. zu denaturieren imstande ist, welche Abbauprodukte nicht mehr allergisierend auf den Menschen wirken, gemäß dem Oberbegriff des Patentanspruchs 1.

### Stand der Technik:

Allergieformen verschiedenster Art sind immer mehr zunehmend, wobei Milben einer der Verursacher dieser Allergien sind. Milben sind überall vorzufinden. Milben ernähren sich von den menschlichen Hautschuppen, welche hauptsächlich aus Keratin-Eiweiß bestehen. Milben als solche verursachen keine Allergie. Das, was die Allergie bei Menschen auslöst, ist der ausgeschiedene Kot der Milben, der größtenteils aus Proteinen (Eiweißen) besteht, nämlich hauptsächlich aus Polypeptiden in Form von Proteinketten besteht. Ebenso können Schimmelpilzsporen von Schimmelpilzen sowie das Keratin von feinsten Tierhaaren wie auch eine Reihe von Pflanzenpollen oder -sporen Allergieen aus lösen.

Bisher werden in diesem Bereich zur Allergiebekämpfung Antimilbenmittel verwendet, die nur die Abtötung der Milben bewirken. Hierzu sind zum Beispiel durch die DE 4202549A1 ein Verfahren und Mittel zur Beseitigung von Hausmilben und deren Überresten in Wohnungseinrichtungen wie auch durch die DE 3430611A1 Mittel zur Abtötung von Hausstaubmilben und dessen Verwendung bekannt geworden. Ebenso sind durch die US 6130253 Terpene in wässrigen Lösungen zum Abtöten u.a. von Milben bekannt geworden.

Durch die US 5679630 ist eine Reinigungskomposition bekannt geworden, welche unter anderen Wirkstoffen zwischen 0,0001% bis 10% Proteaseenzym enthält, das ein N76D/S103A/V104I Subtilisin Variant vom Bacillus lentus subtilisin ist.

Durch die EP 0425018B1 ist des Weiteren als Wäschewaschmittel eine Reinigungszusammensetzung bekannt geworden, welche ein erstes Endoglykosidase-Enzym vom Typ II und als ein zweites Enzym Subtilisin oder mutiertes Subtilisin, aus der Proteasen, Lipasen, Nucleasen, Glykosidasen umfasst, welche von dem ersten Enzym verschieden sind, vorzugsweise einer Exoglykosidase, und Kombinationen hiervon umfassenden Gruppe gewählt wird.

### Technische Aufgabe:

Aufgabe der Erfindung ist es, ein Mittel bzw. eine Wirkstoffkombination einzusetzen, welches bzw. welche den für Menschen allergieauslösenden Milbenkot oder Schimmelpilzsporen wie auch feine Tierhaare oder Pflanzenpollen unschädlich macht.

### Offenbarung der Erfindung sowie deren Vorteile:

Gelöst wird die Aufgabe bei der Verwendung eines gattungsgemäßen Wirkstoffes gemäß dem Oberbegiff des Anspruchs 1 dadurch, dass als Wirkstoff eines der Enzyme Auxillase, Alcalase, Bromelain, alpha-Chymotrypsin, Collagenase, Pepsin, Pronase, Pancreatin, Thrombin oder Trypsin, verwendet wird.

In weiterer vorteilhafter Ausgestaltung der Erfindung besteht der Wirkstoff aus einer Wirkstoffkombination aus einem der verwendeten Enzyme Auxillase, Alcalase, Bromelain, alpha-Chymotrypsin, Collagenase, Pepsin, Pronase, Thrombin, Pancreatin oder Trypsin und Wasser.

In weiterer vorteilhafter Ausgestaltung der Erfindung sind der Wirkstoffkombination aus einem der Enzyme Auxillase, Alcalase, Bromelain, alpha-Chymotrypsin, Collagenase, Pepsin, Pronase, Pancreatin, Thrombin oder Trypsin und Wasser zusätzlich anionische Tenside zugegeben.

In weiterer vorteilhafter Ausgestaltung der Erfindung sind den vorgenannten Wirkstoffkombinationen, nämlich aus einem der Enzyme Auxillase, Alcalase, Bromelain, alpha-Chymotrypsin, Collagenase, Pepsin, Pronase, Pancreatin, Thrombin oder Trypsin und/oder Wasser und/oder anionische Tenside zusätzlich Duftstoffe und/oder Konservierungsmittel zugegeben.

In weiterer vorteilhafter Ausgestaltung der Erfindung können die Enzyme Auxillase, Alcalase, Bromelain, alpha-Chymotrypsin, Collagenase, Pepsin, Pronase, Thrombin, Trypsin in Mischungen von zwei oder mehreren der genannten Enzymen verwendet werden.

In höchst vorteilhafter und bevorzugter Verwendung eines Wirkstoffes zur Inhibierung von Milbenkot und/oder Schimmelpilzsporen sowie des Keratins von Tierhaaren und/oder von Pflanzenpollen wird als Wirkstoff das Enzym Auxillase verwendet, welches die Polypeptide des Milbenkotes, der Schimmelpilzsporen sowie des Keratins von Tierhaaren und/oder von Pflanzenpollen oder -sporen aufspaltet bzw. wenigstens teilweise denaturiert. Vorteilhaft besteht der Wirkstoff aus einer Wirkstoffkombination aus dem Enzym Auxillase und Wasser, wie Laborwasser (Aqua bidest).

Insbesondere durch das Enzym Auxillase werden die Polypeptide des Milbenkotes in Form von Proteinketten in Oligopeptide, wie Mono-, Di- oder Tripeptide, wie auch Schimmelpilzsporen wie auch das Keratin von Tierhaaren und/oder Pflanzenpollen, abgebaut bzw. denaturiert, welche Abbauprodukte nicht mehr allergisierend auf den Menschen wirken. Aber auch andere Proteasen bzw. Enzyme, welche in der Lage sind, die spezifischen Polypeptide des Milbenkotes in Form von Proteinketten abzubauen zu Oligopeptide, Mono-, Di- oder Tripeptide, ebenso wie Schimmelpilzsporen sowie das Keratin von Tierhaaren und/oder Pflanzenpollen, können verwendet werden. Als Proteasen können Endopeptidasen wie auch Exopepdidasen zur Anwendung gelangen.

Bevorzugt hat das Enzym Auxillase oder eines verwendeten Enzyme in der Wirkstoffkombination einen prozentualen Inhalt von 0,1% bis 10%.

In weitere Ausgestaltung der Erfindung hat des Weiteren im Gesamtgehalt der Wirkstoffkombination das Wasser Anteile bevorzugt zwischen 1 % bis 85%. Ebenfalls haben im Gesamtgehalt der Wirkstoffkombination die anionischen Tenside Anteile bevorzugt zwischen 3% -15%. In einer weiteren Ausgestaltung der Erfindung haben im Gesamtgehalt der Wirkstoffkombination die Duftstoffe Anteile bevorzugt zwischen 1 % bis 3 %. In weitere Ausgestaltung der Erfindung haben im Gesamtgehalt der Wirkstoffkombination die Konservierungsmittel bevorzugt Anteile von 0,5% bis 3%.

Die genannten prozentualen Anteile der Wirkstoffkombination können auch für die des Weiteren genannten Enzyme Alcalase, Bromelain, alpha-Chymotrypsin, Collagenase, Pepsin, Pronase, Thrombin oder Trypsin verwendet werden. Damit hat zum Beispiel eines der Enzyme Auxillase, Alcalase, Bromelain, alpha-Chymotrypsin, Collagenase, Pepsin, Pronase, Thrombin, Trypsin, bevorzugt allerdings das Enzym Auxillase, gegebenenfalls auch in Mischungen von zwei oder mehreren der genannten Enzyme, in der Wirkstoffkombination einen prozentualen Inhalt bzw. Anteil von 0,1% bis 10% bzw. hat die Mischung von zwei oder mehreren der genannten Enzyme in der Wirkstoffkombination einen prozentualen Inhalt bzw. Anteil von 0,1% bis 10%.

Sämtliche vorstehenden Angaben von Anteilstoffen in der Wirkstoffkombination sind in Gewichtsprozenten angegeben.

In weitere Ausgestaltung der Erfindung wird die Wirkstoffkombination in Behältnissen zum Sprühen und Spritzen abgefüllt. Des Weiteren kann das Behältnis zum Spritzen ein Nasenspray sein, mittels desselben die Wirkstoffkombination direkt auf die Nasenschleimhäute aufgesprüht werden kann. Oder die Wirkstoffkombination ist in Behältnisse, wie Säckchen, oder in Textilien oder Textilstoffen eingebunden.

Die Wirkstoffkombination kann in flüssiger oder pastöser oder gelartiger Form vorliegen. Beispielsweise kann ein Spray- oder Druckbehälter die Wirkstoffkombination in flüssiger oder pastöser oder gelartiger Form enthalten, so dass die Wirkstoffkombination direkt auf die Nasenschleimhäute durch Sprühen oder Pasten- oder Gelverteilung aufgebracht werden kann.

Insbesondere das Enzym Auxillase ist als Spezificum in der Wirkstoffkombination zur Inhibierung von Milbenkot und/oder von Schimmelpilzsporen einzusetzen, wobei nachfolgend ein Beispiel einer Rezeptur in Gewichtsprozenten mit der Protease Auxillase angegeben ist:

| | |
|---|---|
| Wasser | 1% - 85% |
| Auxillase | 0,1% - 10% |
| Anionische Tenside | 3% - 15% |
| Duftstoffe | 0,1 % - 3% |
| Konservierungsmittel | 0,5% - 3% |

Diese wässrige Wirkstoffkombination kann in Behältnissen zum Besprühen oder Bespritzen abgefüllt sein. Es sind auch andere Anwendungsformen möglich, nämlich zum Beispiel das Einfüllen oder Aufbringen der Wirkstoffkombination in Säckchen, in Textilien oder auf bzw. in Textilstoffen.

Milbenkot ist insbesondere in Matratzen oder Betten zu finden. Nach Besprühen dieser Flächen mit der Wirkstoffkombination wird der Milbenkot nach einer Wirkungszeit einfach abgesaugt. Dieser Vorgang kann bedenkenlos immer wiederholt werden, da das Mittel rein biologisch und für den Menschen unschädlich ist.

### Gewerbliche Anwendbarkeit:

Der Gegenstand der Erfindung ist zur Denaturierung des Keratins von Tierhaaren gewerblich anwendbar und leistet damit einen Beitrag zur Minimierung von Allergien auf Milbenkot, Schimmelpilze, feinste Tierhaare und Pflanzenpollen oder -sporen. Der besondere Vorteil der Erfindung besteht darin, dass das verwendete enzymatische Mittel den im Wesentlichen aus Polypeptiden bestehenden Milbenkot oder Schimmelpilzsporen oder Keratin oder Pflanzenpollen oder - sporen aufspaltet, welche Abbauprodukte nicht mehr allergisierend auf den Menschen wirken.

## Patentansprüche

1. Verwendung eines Wirkstoffes zur Denaturierung des Keratins von Tierhaaren, wobei als Wirkstoff ein solches Enzym verwendet wird, welches die Proteinketten des Keratins von Tierhaaren in Oligopeptide, Mono-, Di- oder Tripeptide, abzubauen bzw. zu denaturieren imstande ist, welche Abbauprodukte nicht mehr allergisierend auf den Menschen wirken, **dadurch gekennzeichnet,**
**dass** als Wirkstoff eines der Enzyme Auxillase, Alcalase, Bromelain, alpha-Chymotrypsin, Collagenase, Pepsin, Pronase, Pancreatin, Thrombin oder Trypsin, verwendet wird.

2. Verwendung eines Wirkstoffes nach Anspruch 1,
**dadurch gekennzeichnet, dass** der Wirkstoff aus einer Wirkstoffkombination aus einem der Enzyme Auxillase, Alcalase, Bromelain, alpha-Chymotrypsin, Collagenase, Pepsin, Pronase, Pancreatin, Thrombin oder Trypsin und Wasser besteht.

3. Verwendung einer Wirkstoffkombination nach Anspruch 2,
**dadurch gekennzeichnet, dass** der Wirkstoffkombination aus einem der vorgenannten Enzyme anionische Tenside und Wasser zugegeben sind.

4. Verwendung einer Wirkstoffkombination nach Anspruch 2 oder 3, **dadurch gekennzeichnet, dass** den vorgenannten Wirkstoffkombinationen der Ansprüche 2 und/oder 3 zusätzlich Duftstoffe und/oder Konservierungsmittel zugegeben sind.

5. Verwendung einer Wirkstoffkombination nach einem der Ansprüche 1 bis 4,
**dadurch gekennzeichnet, dass** die Enzyme Auxillase, Alcalase, Bromelain, alpha-Chymotrypsin, Collagenase, Pepsin, Pronase, Pancreatin, Thrombin, Trypsin in Mischungen von zwei oder mehreren der genannten Enzyme verwendet werden.

6. Verwendung einer Wirkstoffkombination nach einem der Ansprüche 1 bis 5,
**dadurch gekennzeichnet, dass** eines der verwendeten Enzyme Auxillase, Alcalase, Bromelain, alpha-Chymotrypsin, Collagenase, Pepsin, Pronase, Pancreatin, Thrombin, Trypsin, bevorzugt das Enzym Auxillase, in der Wirkstoffkombination einen prozentualen Inhalt bzw. Anteil von 0,1% bis 10% hat.

7. Verwendung einer Wirkstoffkombination nach einem der Ansprüche 1 bis 6,
**dadurch gekennzeichnet, dass** eine Mischung von zwei oder mehreren der verwendeten Enzyme Auxillase, Alcalase, Bromelain, alpha-Chymotrypsin, Collagenase, Pepsin, Pronase, Pancreatin, Thrombin, Trypsin, in der Wirkstoffkombination einen prozentualen Inhalt bzw. Anteil von 0,1% bis 10% hat.

8. Verwendung einer Wirkstoffkombination nach einem der Ansprüche 1 bis 7,
**dadurch gekennzeichnet, dass** im Gesamtgehalt der Wirkstoffkombination das Wasser 1 % bis 85% Anteile hat.

9. Verwendung einer Wirkstoffkombination nach einem der Ansprüche 1 bis 8,
**dadurch gekennzeichnet, dass** im Gesamtgehalt der Wirkstoffkombination die anionischen Tenside 3% -15% Anteile haben.

10. Verwendung einer Wirkstoffkombination nach einem der Ansprüche 1 bis 9,
**dadurch gekennzeichnet, dass** im Gesamtgehalt der Wirkstoffkombination die Duftstoffe 1 % bis 3 % Anteile haben.

11. Verwendung einer Wirkstoffkombination nach einem der Ansprüche 1 bis 10,
**dadurch gekennzeichnet, dass** im Gesamtgehalt der Wirkstoffkombination die Konservierungsmittel 0,5% bis 3% Anteile haben.

12. Verwendung einer Wirkstoffkombination nach Anspruch 1 bis 11,
**dadurch gekennzeichnet, dass** die Wirkstoffkombination in Behältnissen zum Sprühen und Spritzen abgefüllt wird.

13. Verwendung einer Wirkstoffkombination nach Anspruch 1 bis 12,
**dadurch gekennzeichnet, dass** die Wirkstoffkombination in Behältnisse, wie Säckchen, oder in Textilien oder Textilstoffen eingebunden ist.

14. Verwendung einer Wirkstoffkombination nach Anspruch 12,
**dadurch gekennzeichnet, dass** das Behältnis zum Spritzen ein Nasenspray ist zum Aufsprühen der Wirkstoffkombination auf die Nasenschleimhäute.

15. Verwendung einer Wirkstoffkombination nach einem der vorherigen Ansprüche, **dadurch gekennzeichnet, dass** die Wirkstoffkombination in flüssiger oder pastöser oder gelartiger Form vorliegt.
